# EUROPÄISCHE PATENTSCHRIFT

(11) **EP 3 601 210 B1**
(45) Veröffentlichungstag und Bekanntmachung des Hinweises auf die Patenterteilung: **16.12.2020**
(21) Anmeldenummer: 18709302.6
(22) Anmeldetag: 28.02.2018
(51) Int. Cl.: C07C 213/08, C07C 215/10

(54) **KATALYTISCHES VERFAHREN ZUR HERSTELLUNG VON N,N-DIMETHYLGLUCAMIN AUSGEHEND AUS N-METHYLGLUCAMIN**
CATALYTIC PROCESS FOR THE MANUFACTURE OF N,N-DIMETHYLGLUCAMIN DEPARTING FROM N-METHYLGLUCAMIN
PROCEDE CATALYTIQUE POUR LA PREPARATION DE N,N-DIMETHYLGLUCAMINE EN UTILISANT N-METHYLGLUCAMINE

(30) Priorität: 20.03.2017 EP 17161763
(43) Veröffentlichungstag der Anmeldung: 05.02.2020
(73) Patentinhaber: Clariant International Ltd, 4132 Muttenz (CH)
(72) Erfinder: AKGÜN, Ertan, 85049 Ingolstadt (DE); LINK, Martin, 84453 Mühldorf (DE); WERNER, Sarah, 84453 Mühldorf (DE); RAAB, Klaus, 84508 Burgkirchen (DE); KLUG, Peter, 63762 Grossostheim (DE); SCHEITZENEDER, Karl, 84549 Engelsberg (DE); KREUZPOINTNER, Stefan, 84513 Töging (DE)
(74) Vertreter: Mikulecky, Klaus
(86) Internationale Anmeldenummer: PCT/EP2018/054856
(87) Internationale Veröffentlichungsnummer: WO 2018/172025

(56) Entgegenhaltungen:
- EP-A1- 0 614 881
- EP-A1- 0 663 389
- DE-A1- 2 118 283
- GB-A- 908 203

## Beschreibung

Das hier beschriebene N,N-Dimethylglucamin (D-Glucitol, 1-deoxy-1-(dimethylamino)-)ist eine wertvolle Verbindung, welche für eine Vielzahl von kommerziellen Anwendungen geeignet ist. Darunter fällt zum Beispiel der Einsatz als Zusatz in Benetzungsmitteln, Reinigungsmitteln, Weichmachern, Gleit-und Schmiermitteln und Ähnlichem. Neuere Anwendungsgebiete finden sich auch in der Farben- und Lackherstellung, dem Pflanzenschutz, in der Medizin und in der Kosmetik. Das auf nachwachsenden Rohstoffen basierende Produkt kann auch als mildes Neutralisationsmittel oder in protonierter Form als hydrophiles Kation verwendet werden. Für dieses breite Anwendungsspektrum müssen zahlreiche Qualitätsanforderungen eingehalten werden, welche in bekannten Verfahren zur Herstellung von N,N-Dimethylglucamin nicht beschrieben sind.

Das hier beschriebene Verfahren ermöglicht die direkte Herstellung von wenig gefärbten N,N-Dimethylglucamin-Lösungen aus kommerziell gut zugänglichem N-Methylglucamin bei besonders milden Bedingungen mit Hilfe eines verbesserten, zweistufigen Verfahrens ohne aufwändige Aufreinigung.

Zur Herstellung von N,N-Dimethylglucamin sind in der Literatur einige Verfahren beschrieben, allerdings liefern diese einen oder mehrere Nachteile.

EP-0614881 beschreibt die Umsetzung von N-Monoalkylpolyhydroxyverbindung mit Aldehyden im Verhältnis 1 : 0,9 - 1,5 bzw. 1 : 1,1 - 1,03 mit anschließender Hydrierung über einem metallischen Hydrierkatalysator, z. B. Raney-Ni zum tertiären Dialkylpolyhdroxyamin. Diese erfordert einen Wasserstoffdruck von 10 bis 150 bar und Temperaturen von 70 bis 150 °C. Insbesondere wird die Umsetzung in zwei Reaktionsschritten durchgeführt, wobei man
a) zunächst ein sekundäres N-Monoalkylpolyhydroxyamin (z. B. N-Methylglucamin) mit einem Aldehyd (z. B. Formaldehyd) im Molverhältnis von 1 : 0,90 - 1,5, vorzugsweise 1 : 1,03 - 1,1, in Wasser als Lösungsmittel bei einer Temperatur von 15 bis 60 °C, vorzugsweise von 20 bis 40 °C, und Atmosphärendruck zum N-Monoalyklpolyhydroxyamin/Aldehyd-Addukt umsetzt und
b) das im Schritt a) enthaltene Reaktionsprodukt (das im Wesentlichen aus dem gebildeten Addukt und Wasser besteht) in Gegenwart eines metallischen Hydrierkatalysators mit Wasserstoff bei einem Druck von 10 bis 150 bar, vorzugsweise 30 bis 100 bar, und einer Temperatur von 70 bis 150 °C, vorzugsweise 80 bis 130 °C zum tertiären Dialkylpolyhydroxyamin (z. B. N,N-Dimethylglucamin) hydriert.

EP-0614881 beschreibt, dass mit diesem Verfahren hohe Ausbeuten erzielt werden können, allerdings sind keine weiteren Angaben zur Aufarbeitung der rohen Reaktionslösung beschrieben. Die Patentschrift beschreibt die Durchführung der Reaktion in wässriger Lösung, jedoch ist keine Information zu Qualitätsparametern des Dimethylglucamins mit Ausnahme des Schmelzpunktes zu finden. Das bedeutet, dass eine Kristallisation aus der wässrigen Reaktionslösung durchgeführt worden sein muss, die in guten Ausbeuten zum Produkt führt. Dies ist jedoch für die kommerzielle Verwendung des Dimethylglucamines nicht vorteilhaft, da Dimethylglucamin als Feststoff schwieriger als eine Flüssigkeit zu handhaben ist und es vorteilhaft wäre, Synthesebedingungen zu finden, die direkt ohne einen Aufreinigungsschritt zu einer Lösung von Dimethylgucamin in einem wasserhaltigen Medium führt. In diesem Falle sind neben Ausbeute und Schmelzpunkt des umkristallisierten Dimethylglucamins weitere Qualitätsparameter relevant, wie z. B. die Farbe der Reaktionslösung und der Gehalt an Ausgangsverbindugen wie N-Methylglucamin, Formaldehyd oder Nebenprodukten wie Säuren, die ohne Umkristallisation im Produkt verbleiben und dementsprechend limitiert werden müssen.

Andere Verfahren beschreiben einstufige Verfahren, allerdings erfordert z. B. DE-2118283 die Verwendung von Ag-Pd Katalysatoren und ebenfalls hohe Temperaturen von 100 °C bis 250 °C. Die Verwendung eines teuren Katalysators führt zu höheren Produktionskosten.

Des Weiteren ist bekannt, dass bei der Herstellung von tertiären Aminen aus sekundären Aminen mit Formaldehyd und Wasserstoff nur schlechte Ausbeuten von unter 90 % erreicht werden können. EP-0142868B1 beschreibt, dass bessere Ergebnisse erreicht werden können, wenn geträgerte Katalysatoren weiche maximal 10 wt.-% Ni, Co, Ru, Rh Pd or Pt auf Aktivkohle enthalten verwendet werden. Übliche und günstige Hydrierkatalysatoren wie Raney-Ni oder Raney-Co führen zu schlechteren Produktqualitäten. Auch in diesem Verfahren sind lediglich gaschromatographische Reinheiten bestimmt worden.

Um trotzdem diese üblichen Katalysatoren einzusetzen, wird zum Beispiel in GB-908203 vorgeschlagen, die Reaktion ausgehend von 1,3,4,6-tetraacetyl-D-glucosamine zu starten bzw. wasserentziehende Stoffe wie Zeolithe, MgSO₄ oder CaCl₂ (US-4190601) einzusetzen.

EP-A-663 389 lehrt ein Verfahren zur Herstellung von Aminoalkoholen, dadurch gekennzeichnet, dass man Hydroxycarbonylverbindungen mit Wasserstoff und Ammoniak oder einem primären oder sekundären Amin bei Temperaturen von 0 bis 300 °C und Drücken von 1 bis 400 bar in Gegenwart von Katalysatoren, deren katalytisch aktive Masse aus 50 bis 100 Gew.-% Ruthenium besteht, umsetzt.

Die bekannten Verfahren bieten somit nur unzureichende Lösungen für die Herstellung von N,N-Dimethylglucamin-Lösungen mit niedrigen Hazen-Farbzahlen aus N-Methylglucamin. So können die notwendigen Qualitätsparameter, speziell die Farbe der Reaktionslösung oft nicht eingehalten werden (siehe Vergleichsbeispiele) oder die Ausbeuten sind zu gering. Auch führen die bekannten Verfahren zu höheren Herstellkosten, da sie entweder mehrstufige Synthesen, Aufreinigung der rohen Reaktionslösung oder teurere Ausgangssubstanzen (z. B. 1,3,4,6-tetra-acetyl-D-glucosamine oder hochreines N-Methylglucamin) erfordern oder teure Katalysatoren verwendet werden müssen. Ein Recycling der Katalysatoren um Kosten zu sparen ist in den bekannten Verfahren ebenfalls nicht beschrieben.

Daher ist die Bereitstellung eines kostengünstigen, katalytischen Verfahrens zur Herstellung von N,N-Dimethylglucamin-Lösungen ausgehend von kostengünstigem, technischen oder reinem N-Methylglucamin notwendig. Zur Eignung für kommerzielle Anwendungsgebiete wie beispielsweise Farben- und Lackherstellung, Pflanzenschutz, Medizin und Kosmetik muss das nach dem erfindungsgemäßen Verfahren hergestellte N,N-Dimethylglucamin und dessen wässrige Lösungen bestimmte Qualitätsanforderungen in Bezug auf Farbe und maximalen Gehalt an bestimmten Nebenkomponenten erfüllen, die mit den Verfahren des Standes der Technik nicht erreicht werden können.

Es wurde überraschenderweise gefunden, dass bei der Hydrierung der Ausgangsstoffe Formaldehyd und N-Methylglucamin in Gegenwart von Wasserstoff unter Druck und eines Katalysators dann ein verbessertes Produkt erhalten wird, wenn die Hydriertemperatur niedriger als diejenige des Standes der Technik ist.

Gegenstand der Erfindung ist ein Verfahren zur Herstellung von N,N-Dimethyglucamin, indem man zunächst eine wässrige Lösung von N-Methylglucamin mit einer wässrigen Lösung von Formaldehyd bei 15 - 40 °C umsetzt, und anschließend bei einem Druck von 20 - 120 bar und einer Temperatur T = 30 - 68 °C mit Wasserstoff unter Metallkatalyse umsetzt, wobei nach vollendeter Wasserstoffaufnahme bei 20 - 68 °C eine weitere Nachhydrierung bei 70 - 120 bar und bei 70 - 110 °C angeschlossen wird.

Unter diesen Reaktionsbedingungen wurde überraschenderweise gefunden, dass farbarme Dimethylglucamin-Lösungen erhalten werden können.

Alle Prozentangaben sind Gewichtsprozente, es sei denn eine andere prozentuale Grundlage wäre angegeben.

Der Metallkatalysator ist bevorzugt ein ungeträgerter Metallkatalysator. In einer weiteren Ausführungsform enthält der Metallkatalysator Cobalt oder Nickel, bevorzugt Raney-Nickel.

Die Hydrierung erfolgt vorzugsweise bei einem Wasserstoffdruck von 20 - 120 bar, besonders bevorzugt bei 70 - 110 bar.

Die Reaktionstemperatur bei der Hydrierung liegt im erfindungsgemäßen Verfahren vorzugsweise bei T = 20 - 68 °C, bevorzugt 35 - 65 °C, insbesondere bevorzugt 40 - 50 °C.

Im erfindungsgemäßen Verfahren liegt das Molverhältnis N-Methylglucamin : Formaldehyd vorzugsweise bei 1 : 1 bis 1 : 1,5, bevorzugt bei 1 : 1 bis 1 : 1,2, insbesondere bevorzugt bei 1 : 1,01 bis 1 : 1,08.

Das erfindungsgemäße Verfahren erlaubt das Recycling des Hydrierkatalysators. Bevorzugt kann der Hydrierkatalysator mehr als fünfmal wiederverwendet werden, ohne dass die Farbe des Endproduktes signifikant dunkler wird.

Das erfindungsgemäße Verfahren wird bevorzugt in einem gut durchmischten gerührten Reaktor oder in einem Schlaufenreaktor mit externem Umpump und guter Durchmischung durchgeführt. Dieser Reaktor ist temperiert um gegebenenfalls auftretende Exo- oder Endothermien abfangen zu können und die Temperatur über die gesamte Reaktionszeit konstant zu halten.

Stellt man eine Dimethylglucamin-Lösung mit dem erfindungsgemäßen Verfahren her, so beträgt die Hazen-Farbzahl einer resultierenden, 50 % Lösung von N,N-Dimethylglucamin in Wasser weniger als 500, bevorzugt < 300, insbesondere < 200.

Die nach dem erfindungsgemäßen Verfahren hergestellten Lösungen enthalten, bezogen auf eine 50 %ige Dimethylglucaminlösung, weniger als 2 %, bevorzugt weniger als 1 %, besonders bevorzugt < 0.25 % des Ausgangsprodukts N-Methylglucamin.

Die nach dem erfindungsgemäßen Verfahren hergestellten Lösungen enthalten, bezogen auf eine 50 %ige Dimethylglucaminlösung, bevorzugt < 0.1 % Formaldehyd.

Im Anschluss an an das erfindungsgemäße Verfahren wird ein Nachhydrierschritt angeschlossen. Dazu wird nach der vollständigen Zugabe der Formaldehydlösung und beendeter Wasserstoffaufnahme eine Hydrierung bei 60 - 110 °C vorgenommen. Das aus dem erfindungsgemäßen Verfahren erhaltene Produkt wird vor der Nachhydrierung vorzugsweise nicht isoliert.

An das erfindungsgemäße Verfahren kann im Anschluss an das erfindungsgemässe Verfahren ein Destillationsschritt zur Entfernung von überschüssigem Wasser und dem als Nebenprodukt erhaltenen Methanol angeschlossen sein. Hierbei werden Restgehalt von bevorzugt < 0.1 % Methanol erhalten.

Die Konzentration der eingesetzten wässrigen N-Methylglucaminlösung liegt im Bereich von 35 - 70 Gew.-%, bevorzugt 40 - 65 Gew.-%, insbesondere bevorzugt 50 - 60 Gew.-%. Technisches N-Methylglucamin kann im Prinzip nach WO-92/06073 aus Glucosesirup hergestellt werden und fällt ohne weitere Aufreinigung als eine etwa 60 % wässrige Lösung an.

Formaldehyd wird als wässrige Lösung, bevorzugt mit einer Konzentration zwischen 10 und 60 Gew.-%, insbesondere bevorzugt zwischen 30 und 40 Gew.-% verwendet.

Das nach dem erfindungsgemäßen Verfahren hergestellte Produkt liefert die folgenden Qualitätsparameter (bezogen auf eine 50 % Lösung von N,N-Dimethylglucamin in Wasser):
- Die Produkte weisen eine Hazen Farbzahl kleiner 500, bevorzugt kleiner 300; insbesondere kleiner 200 auf. Dies ist zum Beispiel für die Anwendung in der Kosmetik oder in Farben und Lacken entscheidend.
- Der Restgehalt an N-Methylglucamin (Bestimmung mittels GC) ist kleiner 2 Gew.-%, bevorzugt kleiner 1 Gew.-%, insbesondere kleiner 0,25 Gew.-%. Erhöhte Werte würden zu einem höheren Anteil an sekundären Aminen und potentieller Nitrosierbarkeit führen, was für eine Vielzahl an Anwendungen und Formulierungen störend ist.

- Des Weiteren kann die Anwesenheit von kurzkettigen freien sowie chemisch gebundenen Monocarbonsäuren bei verschiedenen Anwendungen durch z. B. Salzbildung stören. Als Leitsubstanz wird hier der Gehalt an Ameisensäure (Bestimmung über Ionenchromatographie (IC)) beschrieben, welcher mit dem erfindungsgemäßen Verfahren unter 1 Gew.-%, bevorzugt unter 0,5 Gew.-%, insbesondere unter 0,1 Gew.-% liegt.
- Der Gehalt an freiem und chemisch gebundenem Formaldehyd (Bestimmung über (PV-LC-114)) liegt mit dem beschriebenen Verfahren unter 0,5 Gew.-%, bevorzugt kleiner 0,1 Gew.-%, insbesondere kleiner 0,05 Gew.-%.
- Das erfindungsgemäße Verfahren liefert Produkt, welches einen geringen Gehalt an unerwünschten Metallverunreinigungen wie z. B. Aluminium, Cobalt oder Nickel aufweist (Bestimmung über ICP-OES). Der Nickelgehalt ist kleiner 50 ppm, bevorzugt kleiner 20 ppm, insbesondere kleiner 10 ppm.
- Für die Anwendung störend sind ebenfalls flüchtige organische Verbindungen wie z. B. Methanol, dessen Gehalt nach angeschlossener Destillation mit dem beschriebenen Verfahren unter 0,7 Gew.-%, bevorzugt unter 0,5 Gew.-% insbesondere bevorzugt unter 0,1 Gew.-% liegt.

Die genannten Katalysatoren (z. B. Raney-Ni) verbleiben nach Filtration im Reaktor und werden für weitere Synthesen verwendet. Dies bietet einen entscheidenden Vorteil gegenüber anderen Verfahren, bei denen der Katalysator nicht oder nur wenige Male wiederverwendet werden kann was zu einem erheblichen Anstieg der Produktionskosten führt. Eventuell auftretende Verluste an Katalysatormaterial durch die Filtration können vor jedem neuen Ansatz ergänzt werden.

Um eventuell in der Reaktionslösung vorhandene flüchtige Komponenten zu entfernen oder die gewünschte Konzentration an Wasser einzustellen, kann die erhaltene Reaktionslösung mittels einer Strippung oder Destillation oder einem ähnlichen dem Fachmann bekannten Verfahren aufgearbeitet werden. Die Strippung kann unter Zugabe von Stickstoff oder Wasser bei Temperaturen zwischen 20 - 100 °C, bevorzugt 30 - 80 °C, insbesondere bevorzugt zwischen 40 und 60 °C bei dem entsprechenden Dampfdruck von Wasser stattfinden.

Das erfindungsgemäße Verfahren führt somit zu Mischungen von N-Methylglucamin und Wasser im Mengenverhältnis z. B. 1 : 99 bis 99 : 1, bevorzugt 30 : 70 bis 90 : 10, insbesondere bevorzugt 45 : 55 bis 80 : 20
Falls für die Anwendung des Produktes noch geringere Konzentrationen an Metallen gewünscht werden, kann eine Aufarbeitung über einen Ionentauscher oder ein ähnliches dem Fachmann bekanntes Verfahren stattfinden.

Des Weiteren kann die nach dem erfindungsmäßigen Verfahren hergestellte N,N-Dimethylglucaminlösung auch als reines, kristallines N,N-Dimethylglucamin hergestellt werden. Dies kann durch dem Fachmann bekannte Aufarbeitungsverfahren, zum Beispiel Abdestillation des Wassers oder/und Umkristallisation aus verschiedenen Lösungsmitteln wie etwa Alkohol oder Alkohol/Wasser Gemische geschehen, und ist für die Anwendung im Pharma- und Medizinbereich von Bedeutung.

Das erfindungsgemäße Verfahren ermöglicht die Produktion von N,N-Dimethylglucamin mit Hilfe eines kostengünstigen und einfachen Verfahrens. Im Vergleich zu bisher bekannten Verfahren (z. B. EP-0614881) führt das Verfahren zu niedrigeren Farbzahlen, damit besserer Produktqualität und somit zu einem breiteren technischen Anwendungsspektrum bei reduzierten Kosten.

Der verwendete nickelhaltige oder cobalthaltige Katalysator führt zu einer Reduzierung der Kosten im Vergleich zu anderen Verfahren welche die Verwendung von Edelmetall-Katalysatoren wie Ru, Ag-Pd oder Pt erfordern. Durch die Möglichkeit den Katalysator für viele Reaktionszyklen wiederzuverwenden, werden die Produktionskosten ebenfalls erheblich gesenkt.

Überraschenderweise konnte im Gegensatz zu US-4190601 gefunden werden, dass die Anwesenheit von Wasser die Reaktion nicht stört. Daher kann dieses als Lösemittel verwendet werden. Dies führt bei Einhaltung der beschriebenen, geeigneten Konzentrationen zu zahlreichen Vorteilen bei der Handhabung wie zum Beispiel einer Erniedrigung der Viskosität und der Vermeidung der Kristallisation des Produktes bei Raumtemperatur bzw. Reaktionstemperatur. Ebenso führt die Verwendung von Wasser als Lösemittel zu einer Reduktion der Kosten, Erniedrigung des Gefahrenpotentials und Vermeidung von toxischen bzw. gefährlichen Abfällen.

Die Einhaltung der für die Anwendung erforderlichen Qualitätsparameter ist bei den im Stand der Technik beschriebenen, bekannten Verfahren nicht ausreichend beschrieben bzw. die Verfahren fürhen zu schlechterer Qualität. Mit dem erfindungsgemäßen Verfahren können alle notwendigen Qualitätsparameter, insbesondere der Farbe unter Verwendung von technischem N-Methylglucamin ohne vorherige Aufarbeitung erreicht werden. Dies führt wiederum zu einer Reduktion der Produktionskosten.

### Beispiele

Die in den Beispielen erzeugten Dimethylglucaminlösungen wurden wie folgt analytisch charakterisiert:
Gardnerfarbzahl und Hazenfarbzahl:
Die klaren und gasblasenfreien, wässrigen Dimethylglucamin-Lösungen wurden in 10 mm-Rechteckküvetten eingefüllt. Die Farbzahlmessungen erfolgten bei Raumtemperatur in einem Farbzahlmessgerät vom Typ LICO 690 der Firma Hach Lange.

Gesamtaminzahl durch Säure-Base-Titration:
Die auf einer Analysenwaage genau eingewogenen Proben wurden in Eisessig gelöst und mit 0,1 molarer Perchlorsäure in Eisessig und einem Titroprozessor der Firma Metrohm titriert.

Trockensubstanz (105 °C/2 Stunden):
Die genau eingewogenen Proben wurden in einem Trockenschrank bei 105 °C zwei Stunden lang durch Abdampfen des Wassers bis zur Gewichtskonstanz getrocknet und nach der Trocknung genau ausgewogen.

Wasser (Karl Fischer-Titration):
Der Wassergehalt der genau eingewogenen Proben wurde nach der Karl Fischer-Titrationsmethode mit Karl Fischer-Solvent und Karl Fischer-Titrant bestimmt.

N-Methylglucamin, N,N-Dimethylglucamin und Sorbitol (GC):
Die Proben wurden mit einem sehr großen Überschuss an Essigsäureanhydrid/Pyridin bei 80°C vollständig acetyliert. Die gaschromatographische Bestimmung des Gehalts an N-Methylglucamin, N,N-Dimethylglucamin und Sorbitol erfolgte auf einer 60 m-Agilent HP-5-Säule mit Decanol als internem Standard und einem FID-Detektor.

### Methanol (GC):

Der Gehalt an Methanol wurde nach einer gaschromatographischen Methode für leicht flüchtige Substanzen mit Isobutanol als internem Standard und WLD-Detektor bestimmt.

### Formaldehyd (frei und chemisch gebunden):

Die Proben wurden zur Freisetzung des chemisch gebundenen Formaldehyds mit wässriger Schwefelsäure in Anwesenheit von 2,4-Dinitrophenylhydrazin erhitzt. Durch die Reaktion des Formaldehyds mit 2,4-Dinitrophenylhydrazin bildete sich 2,4-Dinitrophenylhydrazon. Der Gehalt an 2,4-Dinitrophenylhydrazon wurde anschließend flüssigchromatographisch durch HPLC bestimmt. Der Gehalt an freiem Formaldehyd und chemisch gebundenem Formaldehyd wurde als Summe erfasst.

### Nitrosamine (total NNO):

Die Proben wurden auf den Gesamtgehalt an Nitrosaminen in Anlehnung an die Methode ATNC (apparent total nitrosamine content) untersucht. Unter chemischer Denitrosierung im sauren Milieu wurde aus den Nitrosaminen Stickstoffmonoxid freigesetzt und danach mit einem sehr empfindlichen Stickstoffmonoxidspezifischen Detektor quantitativ bestimmt. Die Berechnung und Gehaltsangabe erfolgte als NNO-Gehalt (>N-N=O mit Molmasse 44 g/mol).

### Nickel (ICP-OES):

Der Gehalt an Nickel in den Proben wurde mittels ICP-OES (inductively coupled plasma optical emission spectrometry; optische Emissionsspektrometrie mittels induktiv gekoppelten Plasmas) in Anlehnung an DIN EN ISO 11885 bestimmt.

### Ameisensäure (frei und chemisch gebunden, IC):

Die Proben wurden im wässrig alkalischen Milieu in der Hitze vorbehandelt, um die als Ester und Amid chemisch gebundene Ameisensäure durch Hydrolyse frei zu setzen. Der Gehalt an bereits vor der alkalischen Hydrolyse vorhandener Ameisensäure oder deren Salze und an Ameisensäure bzw. deren Salze, die erst durch die alkalische Hydrolyse frei gesetzt worden waren, wurde als Summe durch lonenchromatographie (IC) bestimmt.

### Vergleichsbeispiel 1: Beispiel 2 aus EP-0614881

In einem 2 Liter-Glasrundkolben ausgestattet mit Rührer, Thermometer und elektrischer Beheizung wurden bei Normaldruck 1500 g einer 43 %igen wässrigen N-Methylglucamin-Lösung durch Verdünnen einer 60 %igen N-Methylglucaminlösung (Gerdnerfarbzahl 1,7, Hazenfarbzahl 263) hergestellt. Die 43 %ige wässrige N-Methylglucamin-Lösung wurde unter Rühren auf 35 °C erwärmt. Aus einem Tropftrichter wurden unter weiterem Rühren insgesamt 288,7 g einer 36,5 %igen wässrigen Formaldehyd-Lösung innerhalb einer halben Stunde bei 35 °C zugetropft. Die Umsetzung zum N-Methylglucamin-Formaldehyd-Addukt verlief schwach exotherm. Die Gardnerfarbzahl der blassgelben, klaren wässrigen N-Methylglucamin-Formaldehyd-Adduktlösung betrug 0,8 und die Hazenfarbzahl 151.

Unmittelbar nach Ende des Zutropfens wurde aus dem 2 Liter-Glasrundkolben eine Teilmenge der wässrigen N-Methylglucamin-Formaldehyd-Adduktlösung in Höhe von 1300 g entnommen und bei Raumtemperatur in einen 2 Liter-Rührautoklaven eingefüllt.

Der 2 Liter-Rührautoklav war ausgestattet mit Rührer, Heizung, Kühlung, Zuführungen für Wasserstoff und Stickstoff, Temperaturmessung, Druckmessung und Sicherheitsventil. 20,4 g Raney-Nickel wurden unter Stickstoff in den 2 Liter-Rührautoklaven eingebracht.

Der 2 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung und Entspannung wurde die Rührgeschwindigkeit auf 800 Upm eingestellt. Unter weiterem Rühren wurde auf 100 °C erhitzt und danach Wasserstoff zugeführt. Die exotherme Hydrierung erfolgte unter Nachpressen des verbrauchten Wasserstoffs bei 800 Upm, 100 °C und 30 bar Wasserstoffdruck. Nach Ende der erkennbaren Wasserstoffaufnahme wurde noch zwei Stunden bei 100 °C und 30 bar Wasserstoffdruck gerührt. Danach wurde auf 30 °C abgekühlt, entspannt, mit Stickstoff gespült und der 2 Liter-Rührautoklav entleert. Der Raney-NickelKatalysator wurde durch eine Druckfiltration unter Stickstoff abgetrennt. Das flüssige Filtrat war dunkelbraun und hatte die Gardnerfarbzahl 8.

Dieses dunkelbraune Filtrat wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 51 % Wirkstoff und 49 % VE-Wasser eingestellt. Diese Produklösung wurde analysiert. Bis auf die Verluste bei der Entleerung und Aufarbeitung war die Ausbeute an gelöstem N,N-Dimethylglucamin im DMG 50 nahezu quantitativ.

Das erhaltene Produkt war wie folgt charakterisiert:

| | |
|---|---|
| Aussehen bei 20 °C: | klar, flüssig, dunkelbraun |
| Gardnerfarbzahl: | 9,4 |
| Hazenfarbzahl: | nicht messbar, da zu dunkel |
| Gesamtaminzahl (Titration): | 133 mg KOH/g |
| Trockensubstanz (105 °C/2 Stunden): | 51,4 % (m/m) |
| Wasser (Karl-Fischer Titration): | 49 % (m/m) |
| N-Methylglucamin (GC): | 0,91 % (m/m) |
| N,N-Dimethylglucamin (GC): | 44,5 % (m/m) |
| Sorbitol (GC): | 0,6 % (m/m) |
| Methanol (GC): | < 0,1 % (m/m) |
| Formaldehyd (frei und chemisch gebunden, LC): | 0,026 % (m/m) |
| Nitrosamine (total NNO): | < 50 µg/kg |
| Nickel (ICP-OES): | 35 µg/g |
| Ameisensäure (frei und chemisch gebunden) | 0,70 % (m/m) |

### Vergleichsbeispiel 2: nach Beispiel 3 aus EP-0614881

In einem 2 Liter-Glasrundkolben ausgestattet mit Rührer, Thermometer und elektrischer Beheizung wurden bei Normaldruck 1500 g einer 43 %igen wässrigen N-Methylglucamin-Lösung durch Verdünnen einer 60 %igen, technischen N-methylglucaminlösung (Gardnerfarbzahl 1,7, Hazenfarbzahl 263) mit VE-Wasser hergestellt.

Die 43 %ige wässrige N-Methylglucamin-Lösung wurde unter Rühren auf 35 °C erwärmt. Aus einem Tropftrichter wurden unter weiterem Rühren insgesamt 288,7 g einer 36,5 %igen wässrigen Formaldehyd-Lösung innerhalb einer halben Stunde bei 35 °C zugetropft. Die Umsetzung zum N-Methylglucamin-Formaldehyd-Addukt verlief schwach exotherm. Danach wurde die klare, blassgelbe Reaktionsmischung zusätzlich noch eine Stunde bei 35 °C gerührt. Die Gardnerfarbzahl dieser blassgelben wässrigen N-Methylglucamin-Formaldehyd-Adduktlösung betrug 0,8 und die Hazenfarbzahl 162.

Danach wurde aus dem 2 Liter-Glasrundkolben eine Teilmenge der wässrigen N-Methylglucamin-Formaldehyd-Adduktlösung in Höhe von 1300 g entnommen und bei Raumtemperatur in einen 2 Liter-Rührautoklaven eingefüllt.

Der 2 Liter-Rührautoklav war ausgestattet mit Rührer, Heizung, Kühlung, Zuführungen für Wasserstoff und Stickstoff, Temperaturmessung, Druckmessung und Sicherheitsventil. 20,4 g Raney-Nickel wurden unter Stickstoff in den 2 Liter-Rührautoklaven eingebracht.

Der 2 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung und Entspannung wurde die Rührgeschwindigkeit auf 800 Upm eingestellt. Unter weiterem Rühren wurde auf 125 °C erhitzt und danach Wasserstoff zugeführt. Die exotherme Hydrierung erfolgte unter Nachpressen des verbrauchten Wasserstoffs bei 800 Upm, 130 °C und 100 bar Wasserstoffdruck. Nach Ende der erkennbaren Wasserstoffaufnahme wurde noch zwei Stunden bei 130 °C und 100 bar Wasserstoffdruck gerührt. Danach wurde auf 30 °C abgekühlt, entspannt, mit Stickstoff gespült und der 2 Liter-Rührautoklav entleert. Der Raney-NickelKatalysator wurde durch eine Druckfiltration unter Stickstoff abgetrennt. Das flüssige Filtrat war tief dunkelbraun und hatte die Gardnerfarbzahl 13,4.

Dieses tief dunkelbraune Filtrat wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 51 % Wirkstoff und 49 % VE-Wasser eingestellt. Die erhaltene Produktlösung wurde analysiert. Bis auf die Verluste bei der Entleerung und Aufarbeitung war die Ausbeute an gelöstem N,N-Dimethylglucamin im DMG 50 nahezu quantitativ.

Das erhaltene Produkt war wie folgt charakterisiert:

| | |
|---|---|
| Aussehen bei 20 °C: | klar, flüssig, tief dunkelbraun |
| Gardnerfarbzahl: | 16,9 |
| Hazenfarbzahl: | nicht messbar, da zu dunkel |
| Gesamtaminzahl (Titration): | 133 mg KOH/g |
| Trockensubstanz (105 °C/2 Stunden): | 52,6 % (m/m) |
| Wasser (Karl-Fischer Titration): | 49 % (m/m) |
| N-Methylglucamin (GC): | 6,0 % (m/m) |
| N,N-Dimethylglucamin (GC): | 35,9 % (m/m) |
| Sorbitol (GC): | 0,6 % (m/m) |
| Methanol (GC): | < 0,1 % (m/m) |
| Formaldehyd (frei und chemisch gebunden, LC): | 0,012 % (m/m) |
| Nitrosamine (total NNO): | < 50 µg/kg |
| Nickel (ICP-OES): | 29 µg/g |
| Ameisensäure (frei und chemisch gebunden) | 0,90 % (m/m) |

| Beispiel | 1-stufig/ 2-stufig | Hydrierdruck | Hydriertemperatur | Farbe (GFZ/ | NMG | Ni | Formaldehyd |
|---|---|---|---|---|---|---|---|
| | | | [°C] | Hazen) | [%] | [ppm] | [%] |
| 1 (Vgl.) | | 30 | 100 | 9,4/- | 0,91 | 35 | 0,03 |
| 2 (Vgl.) | | 100 | 130 | 16,9/- | 6,0 | 29 | 0,01 |
| 1 | 2-stufig | 30 | 20 - 60, dann 100 | 1,5/246 | 0,16 | 5 | 0,02 |
| 2 | 2-stufig | 100 | 20 - 60, dann 130 | 3,5/485 | 0,29 | 2 | 0,01 |
| 3 | 2-stufig | 100 | 20 - 40, dann 90 | 1,0/158 | 0,12 | 4 | 0,03 |

### Die Beispiele 1 (Vgl.) und 2 (Vgl.) aus D1 entsprechen 1-stufigen Verfahren. Beispiel 1:

In einem 2 Liter-Glasrundkolben ausgestattet mit Rührer, Thermometer und elektrischer Beheizung wurden bei Normaldruck 1500 g einer 43 %igen wässrigen N-Methylglucamin-Lösung durch Verdünnen einer 60 %igen, technischen N-methylglucaminlösung (Gardnerfarbzahl 1,7, Hazenfarbzahl 263) mit VE-Wasser hergestellt.

Die 43 %ige wässrige N-Methylglucamin-Lösung wurde unter Rühren auf 35 °C erwärmt. Aus einem Tropftrichter wurden unter weiterem Rühren insgesamt 288,7 g einer 36,5 %igen wässrigen Formaldehyd-Lösung innerhalb einer halben Stunde bei 35 °C zugetropft. Die Umsetzung zum N-Methylglucamin-Formaldehyd-Addukt verlief schwach exotherm. Die Gardnerfarbzahl der blassgelben, klaren wässrigen N-Methylglucamin-Formaldehyd-Adduktlösung betrug 0,8 und die Hazenfarbzahl 154.

Unmittelbar nach Ende des Zutropfens wurde aus dem 2 Liter-Glasrundkolben eine Teilmenge der wässrigen N-Methylglucamin-Formaldehyd-Adduktlösung in Höhe von 1300 g entnommen und bei Raumtemperatur in einen 2 Liter-Rührautoklaven eingefüllt.

Der 2 Liter-Rührautoklav war ausgestattet mit Rührer, Heizung, Kühlung, Zuführungen für Wasserstoff und Stickstoff, Temperaturmessung, Druckmessung und Sicherheitsventil. 20,4 g Raney-Nickel wurden unter Stickstoff in den 2 Liter-Rührautoklaven eingebracht.

Der 2 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung und Entspannung wurde die Rührgeschwindigkeit auf 800 Upm eingestellt. Bei 20 °C wurde Wasserstoff aufgepresst. Dabei startete die exotherme Hydrierung direkt unter Zufuhr des verbrauchten Wasserstoffs bei 15 - 25 bar Wasserstoffdruck und Erwärmung auf 60 °C, dann war die erkennbare Wasserstoffaufnahme beendet. Danach wurde weiter bis 100 °C erhitzt und dann noch bei 800 Upm, 100 °C und 30 bar Wasserstoffdruck zwei Stunden gerührt. Es wurde auf 30 °C abgekühlt, entspannt, mit Stickstoff gespült und der 2 Liter-Rührautoklav entleert. Der Raney-Nickel-Katalysator wurde durch eine Druckfiltration unter Stickstoff abgetrennt. Das flüssige Filtrat war hellgelb und hatte die Gardnerfarbzahl 0,7 und Hazenfarbzahl 130.

Dieses hellgelbe Filtrat wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 51 % Wirkstoff und 49 % VE-Wasser eingestellt. Diese Mischung wurde analysiert. Bis auf die Verluste bei der Entleerung und Aufarbeitung war die Ausbeute an gelöstem N,N-Dimethylglucamin im Produkt nahezu quantitativ.

Das erhaltene Produkt war wie folgt charakterisiert:

| | |
|---|---|
| Aussehen bei 20 °C: | klar, flüssig, hellgelb |
| Gardnerfarbzahl: | 1,5 |
| Hazenfarbzahl: | 246 |
| Gesamtaminzahl (Titration): | 132 mg KOH/g |
| Trockensubstanz (105°C/2 Stunden): | 50,7 % (m/m) |
| Wasser (Karl-Fischer Titration): | 49 % (m/m) |
| N-Methylglucamin (GC): | 0,16 % (m/m) |
| N,N-Dimethylglucamin (GC): | 45,1 % (m/m), abhängig von der NMG-Qualität |
| Sorbitol (GC): | 0,7 % (m/m) |
| Methanol (GC): | < 0,1 % (m/m) |
| Formaldehyd (frei und chemisch gebunden, LC): | 0,022 % (m/m) |
| Nitrosamine (total NNO): | < 50 µg/kg |
| Nickel (ICP-OES): | 5 µg/g |
| Ameisensäure (frei und chemisch gebunden) (Ionenchromatographie nach alkalischer Hydrolyse, IC) | 0,25 %(m/m) |

### Beispiel 2

In einem 2 Liter-Glasrundkolben ausgestattet mit Rührer, Thermometer und elektrischer Beheizung wurden bei Normaldruck 1500 g einer 43 %igen wässrigen N-Methylglucamin-Lösung durch Verdünnen einer 60 %igen, technischen N-methylglucaminlösung (Gardnerfarbzahl 1,7, Hazenfarbzahl 263) mit VE-Wasser hergestellt.

Die 43 %ige wässrige N-Methylglucamin-Lösung wurde unter Rühren auf 35 °C erwärmt. Aus einem Tropftrichter wurden unter weiterem Rühren insgesamt 288,7 g einer 36,5 %igen wässrigen Formaldehyd-Lösung innerhalb einer halben Stunde bei 35 °C zugetropft. Die Umsetzung zum N-Methylglucamin-Formaldehyd-Addukt verlief schwach exotherm. Danach wurde die klare, blassgelbe Reaktionsmischung zusätzlich noch eine Stunde bei 35 °C gerührt. Die Gardner-Farbzahl dieser blassgelben wässrigen N-Methylglucamin-Formaldehyd-Adduktlösung betrug 0,8 und die Hazenfarbzahl 162.

Unmittelbar nach Ende des Zutropfens wurde aus dem 2 Liter-Glasrundkolben eine Teilmenge der wässrigen N-Methylglucamin-Formaldehyd-Adduktlösung in Höhe von 1300 g entnommen und bei Raumtemperatur in einen 2 Liter-Rührautoklaven eingefüllt.

Der 2 Liter-Rührautoklav war ausgestattet mit Rührer, Heizung, Kühlung, Zuführungen für Wasserstoff und Stickstoff, Temperaturmessung, Druckmessung und Sicherheitsventil. 20,4 g Raney-Nickel wurden unter Stickstoff in den 2 Liter-Rührautoklaven eingebracht.

Der 2 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung und Entspannung wurde die Rührgeschwindigkeit auf 800 Upm eingestellt. Bei 20 °C wurde Wasserstoff aufgepresst. Dabei startete die exotherme Hydrierung direkt unter Zufuhr des verbrauchten Wasserstoffs bei 15 - 25 bar Wasserstoffdruck und Erwärmung auf 60 °C, dann war die erkennbare Wasserstoffaufnahme beendet. Danach wurde weiter bis 130 °C erhitzt und dann noch bei 800 Upm, 130 °C und 100 bar Wasserstoffdruck zwei Stunden gerührt. Es wurde auf 30 °C abgekühlt, entspannt, mit Stickstoff gespült und der 2 Liter-Rührautoklav entleert. Der Raney-Nickel-Katalysator wurde durch eine Druckfiltration unter Stickstoff abgetrennt. Das flüssige Filtrat war hellgelb und hatte die Gardnerfarbzahl 0,3 und Hazenfarbzahl 56.

Dieses hellgelbe Filtrat wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 50 % Wirkstoff und 50 % VE-Wasser eingestellt. Diese Mischung wurde analysiert. Bis auf die Verluste bei der Entleerung und Aufarbeitung war die Ausbeute an gelöstem N,N-Dimethylglucamin im DMG 50 nahezu quantitativ.

Das erhaltene Produkt war wie folgt charakterisiert:

| | |
|---|---|
| Aussehen bei 20 °C: | klar, flüssig, tief gelb |
| Gardnerfarbzahl: | 3,5 |
| Hazenfarbzahl: | 485 |
| Gesamtaminzahl (Titration): | 129 mg KOH/g |
| Trockensubstanz (105 °C/2 Stunden): | 49,7 % (m/m) |
| Wasser (Karl-Fischer Titration): | 50 % (m/m) |
| N-Methylglucamin (GC): | 0,29 % (m/m) |
| N,N-Dimethylglucamin (GC): | 43,5 % (m/m) |
| Sorbitol (GC): | 0,6 % (m/m) |
| Methanol (GC): | < 0,1 % (m/m) |
| Formaldehyd (frei und chemisch gebunden, LC): | 0,0097 % (m/m) |
| Nitrosamine (total NNO): | < 50 µg/kg |
| Nickel (ICP-OES): | 2 µg/g |
| Ameisensäure (frei und chemisch gebunden) (Ionenchromatographie nach alkalischer Hydrolyse, IC) | 0,18 % (m/m) |

### Beispiel 3:

In einem 2 Liter-Glasrundkolben ausgestattet mit Rührer, Thermometer und elektrischer Beheizung wurden bei Normaldruck 1500 g einer 43 %igen wässrigen N-Methylglucamin-Lösung durch Verdünnen einer 60 %igen, technischen N-methylglucaminlösung (Gardnerfarbzahl 1,7, Hazenfarbzahl 263) mit VE-Wasser hergestellt.

Die 43 %ige wässrige N-Methylglucamin-Lösung wurde unter Rühren auf 35 °C erwärmt. Aus einem Tropftrichter wurden unter weiterem Rühren insgesamt 288,7 g einer 36,5 %igen wässrigen Formaldehyd-Lösung innerhalb einer halben Stunde bei 35 °C zugetropft. Die Umsetzung zum N-Methylglucamin-Formaldehyd-Addukt verlief schwach exotherm. Die Gardnerfarbzahl der blassgelben, klaren wässrigen N-Methylglucamin-Formaldehyd-Adduktiösung betrug 0,8 und die Hazenfarbzahl 154.

Unmittelbar nach Ende des Zutropfens wurde aus dem 2 Liter-Glasrundkolben eine Teilmenge der wässrigen N-Methylglucamin-Formaldehyd-Adduktlösung in Höhe von 1300 g entnommen und bei Raumtemperatur in einen 2 Liter-Rührautoklaven eingefüllt.

Der 2 Liter-Rührautoklav war ausgestattet mit Rührer, Heizung, Kühlung, Zuführungen für Wasserstoff und Stickstoff, Temperaturmessung, Druckmessung und Sicherheitsventil. 20,4 g Raney-Nickel wurden unter Stickstoff in den 2 Liter-Rührautoklaven eingebracht.

Der 2 Liter-Rührautoklav wurde verschlossen. Dreimal wurde 10 bar Stickstoff aufgedrückt und jeweils entspannt. Danach wurde dreimal 10 bar Wasserstoff aufgedrückt und jeweils entspannt. Nach der Dichtigkeitsprüfung und Entspannung wurde die Rührgeschwindigkeit auf 800 Upm eingestellt. Bei 20 °C wurde Wasserstoff aufgepresst. Dabei startete die exotherme Hydrierung direkt unter Zufuhr des verbrauchten Wasserstoffs bei 15 - 25 bar Wasserstoffdruck. Durch Kühlung wurde der Autoklav dabei in einem Temperaturbereich von 30 - 40 °C gehalten, bis die erkennbare Wasserstoffaufnahme beendet war. Danach wurde weiter bis 90 °C erhitzt und dann noch bei 800 Upm, 90 °C und 30 bar Wasserstoffdruck zwei Stunden gerührt. Es wurde auf 30 °C abgekühlt, entspannt, mit Stickstoff gespült und der 2 Liter-Rührautoklav entleert. Der Raney-Nickel-Katalysator wurde durch eine Druckfiltration unter Stickstoff abgetrennt. Das flüssige Filtrat war hellgelb und hatte die Gardnerfarbzahl 0,6 und Hazenfarbzahl 110.

Dieses hellgelbe Filtrat wurde in einem Rotationsverdampfer bei 60 °C und einem Druck von 20 mbar andestilliert. Das Destillat enthielt vor allem Wasser, wenig Methanol und Spuren von anderen Leichtsiedern. Das andestillierte, viskose N,N-Dimethylglucamin wurde durch Zugabe von VE-Wasser unter guter Durchmischung bei 60 °C auf einen Gehalt von 51 % Wirkstoff und 49 % VE-Wasser eingestellt. Diese Mischung wurde analysiert. Bis auf die Verluste bei der Entleerung und Aufarbeitung war die Ausbeute an gelöstem N,N-Dimethylglucamin im Produkt nahezu quantitativ.

Das erhaltene Produkt war wie folgt charakterisiert:

| | |
|---|---|
| Aussehen bei 20 °C: | klar, flüssig, hellgelb |
| Gardnerfarbzahl: | 1,0 |
| Hazenfarbzahl: | 158 |
| Gesamtaminzahl (Titration): | 131 mg KOH/g |
| Trockensubstanz (105°C/2 Stunden): | 50,8 % (m/m) |
| Wasser (Karl-Fischer Titration): | 49,1 % (m/m) |
| N-Methylglucamin (GC): | 0,12 % (m/m) |
| N,N-Dimethylglucamin (GC): | 44,6 % (m/m) |
| Sorbitol (GC): | 0,6 % (m/m) |
| Methanol (GC): | < 0,1 % (m/m) |
| Formaldehyd (frei und chemisch gebunden, LC): | 0,025 % (m/m) |
| Nitrosamine (total NNO): | < 50 µg/kg |
| Nickel (ICP-OES): | 4 µg/g |
| Ameisensäure (frei und chemisch gebunden) (Ionenchromatographie nach alkalischer Hydrolyse, IC) | 0,21 % (m/m) |

## Patentansprüche

1. Verfahren zur Herstellung von N,N-Dimethyglucamin, indem man eine wässrige Lösung von N-Methylglucamin zunächst mit einer wässrigen Lösung von Formaldehyd bei 15 - 40 °C umsetzt, und anschließend bei einem Druck von 20 - 120 bar und einer Temperatur T = 30 - 68 °C mit Wasserstoff unter Metallkatalyse umsetzt, wobei nach vollendeter Wasserstoffaufnahme bei 20 - 68 °C eine weitere Nachhydrieriung bei 70 - 120 bar und bei 70 - 110 °C angeschlossen wird.

2. Verfahren nach Anspruch 1, wobei der Metallkatalysator Raney-Nickel ist.

3. Verfahren nach Anspruch 1 und/oder 2, das bei einem Wasserstoffdruck von 70 - 110 bar durchgeführt wird.

4. Verfahren nach einem oder mehreren der Ansprüche 1 - 3, wobei die Hydrierung bei 35 - 65 °C, besonders bevorzugt bei 40 - 50 °C durchgeführt wird.

5. Verfahren nach einem oder mehreren der Ansprüche 1 - 4, wobei das Molverhältnis N-Methylglucamin zu Formaldehyd 1 : 1 bis 1 : 1,5, bevorzugt 1 : 1 bis 1 : 1,2, insbesondere bevorzugt 1 : 1,01 bis 1 : 1,08 beträgt.

6. Verfahren nach einem oder mehreren der Ansprüche 1 - 5, wobei der Hydrierkatalysator mehr als 5 mal wiederverwendet wird.

7. Verfahren nach einem oder mehreren der Ansprüche 1 - 6, wobei in einem Rührreaktor oder Loopreaktor umgesetzt wird.

8. Verfahren nach einem oder mehreren der Ansprüche 1 - 7, wobei der verbleibende Gehalt an N-Methylglucamin < 2, bevorzugt < 1, insbesondere < 0,25 Gew.-% beträgt.

9. Verfahren nach einem oder mehreren der Ansprüche 1 - 8, wobei der Rest-Formaldehydgehalt < 0.1 Gew.- % beträgt.

10. Verfahren nach einem oder mehreren der Ansprüche 1 - 9, worin die Hazen-Farbzahl der erhaltenen Lösung von N,N-Dimethylglucamin < 500 ist.

## Claims

1. Process for preparing N,N-dimethylglucamine by reacting an aqueous solution of N-methylglucamine first with an aqueous solution of formaldehyde at 15 - 40°C and subsequently, at a pressure of 20 - 120 bar and a temperature T = 30 - 68°C, with hydrogen under metal catalysis, wherein, after absorption of hydrogen has completed at 20 - 68°C, a further, after-hydrogenation is added at 70 - 120 bar and at 70 - 110°C.

2. Process according to Claim 1, wherein the metal catalyst is Raney nickel.

3. Process according to Claim 1 and/or 2, which is carried out at a hydrogen pressure of 70 - 110 bar.

4. Process according to one or more of Claims 1 - 3, wherein the hydrogenation is carried out at 35 - 65°C, more preferably at 40 - 50°C.

5. Process according to one or more of Claims 1 - 4, wherein the molar ratio of N-methylglucamine to formaldehyde is 1 : 1 to 1 : 1.5, preferably 1 : 1 to 1 : 1.2, especially preferably 1 : 1.01 to 1 : 1.08.

6. Process according to one or more of Claims 1 - 5, wherein the hydrogenation catalyst is reused more than 5 times.

7. Process according to one or more of Claims 1 - 6, wherein reaction takes place in a stirred reactor or loop reactor.

8. Process according to one or more of Claims 1 - 7, wherein the remaining N-methylglucamine content is < 2, preferably < 1, more particularly < 0.25 wt%.

9. Process according to one or more of Claims 1 - 8, wherein the residual formaldehyde content is < 0.1 wt%.

10. Process according to one or more of Claims 1 - 9, in which the Hazen colour number of the resulting solution of N,N-dimethylglucamine is < 500.

## Revendications

1. Procédé pour la préparation de N,N-diméthylglucamine, dans lequel on transforme une solution aqueuse de N-méthylglucamine tout d'abord avec une solution aqueuse de formaldéhyde à une température de 15 à 40 °C, et ensuite avec de l'hydrogène sous catalyse par un métal à une pression de 20 à 120 bars et une température T = 30 à 68 °C, où, après l'absorption d'hydrogène terminée à une température de 20 à 68 °C, une post-hydrogénation supplémentaire à une pression de 70 à 120 bars et une température de 70 à 110 °C est associée.

2. Procédé selon la revendication 1, le catalyseur métallique étant le nickel de Raney.

3. Procédé selon la revendication 1 et/ou la revendication 2, qui est mis en œuvre à une pression d'hydrogène de 70 à 110 bars.

4. Procédé selon l'une ou plusieurs des revendications 1 à 3, l'hydrogénation étant mise en œuvre à une température de 35 à 65 °C, particulièrement préférablement de 40 à 50 °C.

5. Procédé selon l'une ou plusieurs des revendications 1 à 4, le rapport molaire de N-méthylglucamine sur formaldéhyde étant de 1:1 à 1:1,5, préférablement 1:1 à 1:1,2, en particulier préférablement 1:1,01 à 1:1,08.

6. Procédé selon l'une ou plusieurs des revendications 1 à 5, le catalyseur d'hydrogénation étant réutilisé plus de 5 fois.

7. Procédé selon l'une ou plusieurs des revendications 1 à 6, la transformation ayant lieu dans un réacteur agité ou un réacteur à boucle.

8. Procédé selon l'une ou plusieurs des revendications 1 à 7, la teneur restante en N-méthylglucamine étant < 2, préférablement < 1, en particulier < 0,25 % en poids.

9. Procédé selon l'une ou plusieurs des revendications 1 à 8, la teneur résiduelle en formaldéhyde étant < 0,1 % en poids.

10. Procédé selon l'une ou plusieurs des revendications 1 à 9, dans lequel l'indice de couleur Hazen de la solution de N,N-diméthylglucamine obtenue est < 500.
